# EUROPEAN PATENT APPLICATION

(11) **EP 4 809 991 A1**
(43) Date of publication of application: **23.09.2026**
(21) Application number: 25164573.5
(22) Date of filing: 18.03.2025
(51) Int. Cl.: A61F 13/494, A61F 13/495, A61F 13/49

(54) **ABSORBENT ARTICLE WITH POCKET(S)**

(71) Applicant: Drylock Technologies NV, 9240 Zele (BE)
(72) Inventor: DERYCKE, Tom, 9240 Zele (BE); SMET, Steven, 9240 Zele (BE)
(74) Representative: Arnold & Siedsma

(57) **Abstract**

An absorbent article comprising a liquid pervious topsheet, a liquid impervious backsheet, and an absorbent core which comprises absorbent material and which is positioned between the liquid pervious topsheet and the liquid impervious backsheet; said absorbent article having a first and second longitudinal edge and a first and second transverse edge; wherein the absorbent article comprises a waist pocket positioned near the first transverse edge. The absorbent article may further have a second waist pocket. The waist pocket includes hydrophilic nonwoven(s).

## Description

### TECHNICAL FIELD

The present invention pertains to the technical field of absorbent articles, more preferably disposable personal care articles such as diapers, baby pants, adult incontinent garments, and the like. More specifically the present invention relates to an absorbent article, preferably of the diaper-type, comprising a liquid pervious topsheet, a liquid impervious backsheet, and an absorbent core which comprises absorbent material and which is positioned between the liquid pervious topsheet and the liquid impervious backsheet. The absorbent article has a first longitudinal edge and second longitudinal edge and a first and second transverse, wherein the absorbent article comprises a waist pocket positioned near the first transverse edge and/or near the second transverse edge. One or two waist pockets (e.g. pee & poo pockets) may be included at the front and the back of the article.

### BACKGROUND

Absorbent articles are described for example in patent EP 2 900 191 B1 and international patent application WO 2017/158185 A1 in the name of the applicant, which are incorporated herein by reference.

It is further known to provide a guard pocket to further prevent leakage of bodily exudates towards a back of a wearer. Typically, such guard pocket is provided by the provision of additional layers or sheets of material which are conveniently attached to the rear section of the absorbent article. Such a guard pocket is described in WO 2024/141573 in the name of the applicant, which is incorporated herein by reference.

### SUMMARY OF THE INVENTION

Embodiments of the invention aim to provide improved containment of bodily exudates to avoid leakage.

Thereto, a first aspect provides an absorbent article comprising a liquid pervious topsheet, a liquid impervious backsheet, and an absorbent core which comprises absorbent material and which is positioned between the liquid pervious topsheet and the liquid impervious backsheet; said absorbent article having a first and second longitudinal edge and a first and second transverse edge; wherein the absorbent article comprises a waist pocket positioned near the first transverse edge,
wherein the waist pocket comprises
a proximal transversal portion extending from a proximal transverse edge of the waist pocket, which is proximal to the first transverse edge, towards a distal transversal edge of the waist pocket opposite to the proximal transverse edge;
a distal transversal portion extending from the distal transverse edge of the waist pocket towards the proximal transverse edge of the waist pocket; and
a central transversal portion between the proximal transversal portion and the distal transversal portion and comprising a transversal center line of the waist pocket;
a first longitudinal side edge,
a second longitudinal side edge,
a first nonwoven layer and second nonwoven layer; and
preferably at least one elastic material comprising an elastic member positioned between the first and second nonwoven layer and extending substantially transversally between the first longitudinal side edge and the second longitudinal side edge; and
wherein the proximal transversal portion is at least partially attached to a body facing side of the absorbent article and the distal transversal portion is at least partially unattached to the body facing side of the absorbent article,
such that a waist pocket opening is formed between the distal transversal portion and the topsheet; and wherein
the first nonwoven layer and/or the second nonwoven layer comprised by the waist pocket are chosen from hydrophilic nonwovens.

Inventive insights have provided that by using a hydrophilic nonwovens (as compared to hydrophobic nonwovens), bodily exudates may be better contained in the pocket. As a result, and advantageously, leakage prevention may be further improved. The pocket may be arranged at the front of the article (e.g. as a pee pocket) or may be a arranged at the back of the article (e.g. as a poo pocket), the combination of having both pockets (pee & poo) is also possible. Further, by including the elastic material positioned between the first and second nonwoven layer and extending substantially transversally between the first longitudinal side edge and the second longitudinal side edge, the containment performance may be even further increased, namely bodily exudates may "enter" at the partially unattached region while the "escape" is further prevented by aid of the elastic member. Such "escape" prevention is even more significant when the first nonwoven layer and/or the second bodily layer comprises by the waist pocket are chosen from hydrophilic nonwovens because the bodily exudates typically include a great share of water which better "stick" to the hydrophilic nonwoven. Accordingly, the hydrophilic nonwovens of the pocket (having a strong affinity for water") better contain the bodily exudates.

According to an embodiment, the first nonwoven layer and the second nonwoven layer are the same nonwoven connected via a folded connection. The folded connection may be advantageous for quality and feel of a wearer, and can avoid delamination. According to another embodiment, the first nonwoven layer and the second nonwoven layer are different nonwovens and wherein the second nonwoven is an inner nonwoven facing inwards, e.g. facing towards the absorbent core, and wherein the first nonwoven is an outer nonwoven intended to face towards a wear. By using a first nonwoven layer and the second nonwoven layer that are different nonwovens, the pocket performance and production can be finetuned as desired. Preferably, at least the inner nonwoven facing inwards is chosen from a hydrophilic nonwoven, preferably a hydrophilic nonwoven as described herein.

Preferably, the hydrophilic nonwoven(s) of the pocket are chosen from treated nonwovens, such a treated nonwoven comprising polyethylene (PE), polypropylene (PP), polyester or polyethylene terephthalate (PET) or combinations thereof; or wherein the hydrophilic nonwovens are chosen from nonwovens which substantially consist of hydrophilic materials; or wherein the hydrophilic nonwovens are chosen from nonwovens comprising a mixture of hydrophobic polymers blended or grafted with hydrophilic additives.

By using treated nonwovens, useful materials such as polyethylene (PE), polypropylene (PP), polyester or polyethylene terephthalate (PET) or combinations therefor (e.g. as mono or bicomponent nonwovens) can be used, by having said nonwovens treated to be hydrophilic, the performance of the pocket to contain bodily exudates may better in the pocket can be increased, in particular in case at least an inner nonwoven (on the inside of the pocket) is treated to be hydrophilic. Alternatively, the hydrophilic nonwovens are chosen from nonwovens which substantially consist of hydrophilic materials, in particular materials that are originally hydrophilic (not treated to become hydrophobic), examples of nonwovens made from non-treated materials that are originally hydrophilic are cotton and rayon.

According to an embodiment, the hydrophilic nonwoven(s) of the pocket (i.e. the first and/or the second nonwoven) are chosen from nonwovens comprising a mixture of hydrophobic polymers (e.g. PE, PP, PET or combinations thereof) blended or grafted with hydrophilic additives. For example, hydrophilic monomers (e.g., acrylic acid, maleic anhydride) can be chemically grafted onto PP fibers to increase their water absorption. As a result, a nonwoven made therefrom and used for the pocket may cause the pocket to better contain bodily exudates.

According to an embodiment, the hydrophilic nonwoven(s) of the pocket (i.e. the first and/or the second nonwoven) are chosen from treated nonwovens which have been treated with a surface treatment chosen from a surface coating, such as a surfactant coating, a plasma treatment, a corona discharge treatment, preferably a surface coating.

The surface treatments, including the surface coating, plasma treatment (creating functional groups attracting water), and corona discharge treatment (for introducing polar groups) can be used to improve the water affinity of the respective component in order to enhance a desired interaction with bodily exudate.

Any suitable surface coating may be used. The surface coating may be surfactant coating whereby surface active agents are applied to the fibers during of after being processed into a nonwoven.

Preferably, the hydrophilic nonwoven(s) of the pocket (i.e. the first and/or the second nonwoven) are chosen from the group (group A) consisting of:
- a nonwoven which have been treated with a surfactant coating,
- a nonwoven which comprises a coating comprising hydrophilic agent,
- a nonwoven obtained by mixing a hydrophilic agent (e.g. Polyethylene oxide (PEO), polyvinyl alcohol (PVA), or hydrophilic copolymers) directly in a thermoplastic polymer resin (e.g. PP, PE, PET) before being processed into the nonwoven, e.g. via spunbonding, melt blowing, dry-laying, wet-laying, spunlacing or hydroentanglement.

The nonwoven can be treated with a surfactant coating wherein surfactants are applied as a surface coating to lower water's surface tension, allowing absorption. The surfactants may be applied via spray coating, padding, dipping or roller application. The surfactants may be nonionic or ionic. The nonwoven may be treated with a coating comprising hydrophilic agent (e.g. a polymer containing hydrophilic functional groups (e.g., -OH, -COO-), such as PVA or PEG or acrylic coatings. The coating may be applied via dip coating, spray, or the coating may be fixed to the nonwoven with UV or heat curing. In this manner, the durability may be increased.

When referring to a treated component or material (e.g. treated spunbond-meltblown-spunbond, a treated SMS nonwoven, treated polypropylene, treated polylactic acid, and treated biobased polyethylene, treated polypropylene), it is to be understood that such treatment can include one or more of the following treatments: surface coating, plasma treatment (creating functional groups attracting water), and corona discharge treatment (for introducing polar groups) or combinations thereof.

Understandably, the nonwovens chosen from group A may be further treated with a plasma treatment. The plasma treatment may be used as an ecological surface treatment. Plasma treatment can be used to modify the surface properties of nonwovens for enhancement of surface energy, thereby improving wettability and adhesion properties of nonwovens. Consequently, and generally applicable to hydrophilic nonwovens, when included as the nonwoven(s) of the pocket (first and/or second), the adhesion properties can be improved which improves containment of bodily exudates. A preferred example of a hydrophilic nonwovens is a nonwoven chosen from a nonwoven fabric comprising polyolefin in which liquid transport is recognized within 10 seconds as measured by the liquid transport speed specified in EDANA 150 2-93 after heating treatment for an hour at 60° C.

According to an embodiment, the absorbent article is chosen from a diaper, such as a resealable diaper, or diaper pants, such as baby pants, and wherein the first transverse edge is intended to be positioned at the back of a wearer when the article is worn, and wherein the proximal transverse edge corresponds to the first transverse edge, preferably wherein the diaper further comprises a second waist pocket positioned near the second transverse edge.

Preferably, the at least one elastic member of the waist pocket is positioned such that the central grammage of elastic material in the central transversal portion is lower than a proximal grammage of elastic material in the proximal transversal portion.

Preferably, the distal transversal portion comprises a first distal transversal sub-portion extending from the distal transverse edge of the waist pocket and a second distal transversal sub-portion between the first transversal sub-portion and the central transversal portion, and wherein a first distal grammage of elastic material in the first distal transversal sub-portion is higher than a second distal grammage of elastic material in the second distal transversal sub-portion.

Preferably, the distal proximal transversal portion comprises a first proximal transversal sub-portion extending from the proximal transverse edge of the waist pocket and a second proximal transversal sub-portion between the first proximal transversal sub-portion and the central transversal portion, and wherein a first grammage of elastic material in the first proximal transversal sub-portion is higher than a second grammage of elastic material in the second proximal transversal sub-portion.

Preferably, the distal proximal transversal portion and the distal transversal portion has a surface area which is substantially double of a surface area of the central transversal portion.

Preferably, the distal absorbent article comprises a pair of longitudinally extending leg cuffs, wherein the first longitudinal side edge and the second longitudinal side edge of the waist pocket are adhesively attached to the leg cuffs.

Preferably, the distal proximal transverse edge of the waist pocket is adhesively attached to the topsheet.

According to an embodiment, the distal first and second nonwoven layer consist of the same hydrophilic nonwoven material. Preferably, the distal first and second nonwoven layer consist of an at least partially folded hydrophilic nonwoven sheet, preferably wherein the at least partially folded nonwoven sheet is folded according to a C-fold or L-fold configuration. Preferably, a free portion of the at least partially folded nonwoven is facing the body facing side of the absorbent article, and wherein the free portion is hydrophilic.

Preferably, the at least one elastic member is adhesively attached between the first and second nonwoven layer.

Preferably, the first and/or second nonwoven layer comprises a treated spunbond nonwoven, a treated meltblown nonwoven, a treated spunlaced nonwoven or combination thereof. In particular, the treated spunbond nonwoven, a treated meltblown nonwoven, a treated spunlaced nonwoven or combination thereof are treated to render the nonwoven hydrophilic, for example treated according to any of the treatments as described herein.

Preferably, the first and/or second nonwoven layer is a treated spunbond-meltblown-spunbond, a treated SMS nonwoven.

Preferably, the first and/or second nonwoven layer has a grammage of between 4 - 15 gsm, preferably between 6 - 12 gsm, most preferably between 8 - 10 gsm.

Preferably, the first and/or second nonwoven layer comprise, preferably consist of, any one of treated polypropylene, treated polylactic acid, and treated biobased polyethylene.

Preferably, the first and the second nonwoven layer consist of treated polypropylene. The polypropylene may be treated with a surfactant coating, a nonwoven which comprises a coating comprising hydrophilic agent, or nonwovens obtained by mixing a hydrophilic agent directly in a thermoplastic polymer resin before being processed into the nonwoven, e.g. via spunbonding, melt blowing, dry-laying, wet-laying, spunlacing or hydroentanglement.

Preferably, the at least one elastic member comprises a plurality of elastic members which are mutually positioned at a distance of between 4 and 10 mm of each other.

Preferably, the central transversal portion is free of elastic members.

Preferably, the proximal transversal portion is free of elastic members.

According to an embodiment, a second waist pocket may be provided for additional leakage protection. In said embodiment, the first transverse edge is a front transverse edge of the absorbent article, and wherein the waist pocket is a front waist pocket, or wherein the first transverse edge is a rear transverse edge of the absorbent article, and wherein the waist pocket is a rear waist pocket, preferably wherein the absorbent article comprises a second waist pocket positioned near the second transverse edge. Preferably, the second waist pocket comprises
a proximal transversal portion extending from a proximal transverse edge of the second waist pocket, which is proximal to the second transverse edge, towards a distal transversal edge of the second waist pocket opposite to the proximal transverse edge;
a distal transversal portion extending from the distal transverse edge of the second waist pocket towards the proximal transverse edge of the second waist pocket; and
a central transversal portion between the proximal transversal portion and the distal transversal portion and comprising a transversal center line of the second waist pocket;
a first longitudinal side edge,
a second longitudinal side edge,
a first and second nonwoven layer; and
at least one elastic material comprising elastic member positioned between the first and second nonwoven layer and extending substantially transversally between the first longitudinal side edge and the second longitudinal side edge,
wherein the proximal transversal portion is at least partially attached to the body facing side of the absorbent article and the distal transversal portion is at least partially unattached to the body facing side of the absorbent article, such that a second waist pocket opening is formed between the distal transversal portion of the second waist pocket and the topsheet; and
wherein
the first nonwoven layer and/or the second nonwoven layer (of the second pocket) are chosen from a hydrophilic nonwoven.

The first nonwoven layer and/or the second nonwoven layer may be configured as described herein in relation to the (first) pocket.

Preferably, the at least one elastic member of the second waist pocket is positioned such that a central grammage of elastic material in the central transversal portion is lower than a distal grammage of elastic material in the distal transversal portion.

A second aspect of the invention shall now be discussed by aid of the following clauses. By using hydrophilic outer surface, preferably provided by a hydrophilic nonwoven in the respective pocket (front guard and/or rear guard pocket), the containment performance of the pocket can be improved.
Clause 1. An absorbent article comprising an elastic front section, an elastic rear section, and an absorbent body extending in a longitudinal direction between the elastic front section and the elastic rear section,
   wherein the elastic front section and the elastic rear section each comprise a laminate consisting of an inner sheet, an outer sheet, and at least one elastic member positioned between the inner sheet and the outer sheet;
   wherein the absorbent body comprises a liquid pervious topsheet, a liquid impervious backsheet, and an absorbent core positioned between the topsheet and backsheet;
   wherein the elastic rear section comprises a rear folded portion wherein the laminate of the elastic rear section is folded from a rear waistline of the elastic rear section towards the topsheet of the absorbent body; and
   wherein the rear folded portion extends from the rear waistline to a free edge of the rear folded portion to form a rear guard pocket having a pocket opening defined between the free edge of the rear folded portion and the topsheet of the absorbent body,
   wherein
   the rear guard pocket comprises an hydrophilic inner and/or outer surface, preferably provided by a hydrophilic nonwoven, for example a hydrophilic nonwoven which forms the inner and/or outer sheet of the laminate.
Clause 2. The absorbent article according to clause 1, wherein the hydrophilic nonwoven is chosen from a treated nonwoven, such a treated nonwoven comprising polyethylene (PE), polypropylene (PP), polyester or polyethylene terephthalate (PET) or combinations thereof; or wherein the hydrophilic nonwoven is chosen from a nonwoven which substantially consist of a hydrophilic material(s); or wherein the hydrophilic nonwovens are chosen from nonwovens comprising a mixture of hydrophobic polymers blended or grafted with hydrophilic additives, wherein the rear folded portion comprises a first elastic member in proximity of and substantially parallel with the free edge, and a first attachment zone wherein the inner sheet and outer sheet are attached to each other, wherein the first attachment zone is positioned in between the free edge and the first elastic member.
Clause 3. The absorbent article according to the preceding clause, wherein a maximum distance between the first elastic member and the free edge is less than 15mm, preferably less than 12mm, more preferably less than 9mm, and most preferably less than 6mm.
Clause 4. The absorbent article according to any one of the preceding clauses, wherein the hydrophilic nonwovens are chosen from treated nonwovens which have been treated with a surface treatment chosen from a surface coating, such as a surfactant coating, a plasma treatment, a corona discharge treatment, preferably a surface coating, preferably wherein the rear folded portion comprises a second attachment zone wherein the inner sheet and outer sheet are attached to each other, and wherein the second attachment zone is positioned in between the first elastic member and the rear waistline.
Clause 5. The absorbent article according to the preceding clause, wherein the rear folded portion comprises at least one second elastic member positioned between the first elastic member and the second attachment zone.
Clause 6. The absorbent article according to any one of the preceding clauses, wherein the hydrophilic nonwoven is chosen from the group consisting of: a nonwoven which has been treated with a surfactant coating, a nonwoven which comprises a coating comprising hydrophilic agent, or nonwovens obtained by mixing a hydrophilic agent directly in a thermoplastic polymer resin before being processed into the nonwoven, e.g. via spunbonding, melt blowing, dry-laying, wet-laying, spunlacing or hydroentanglement, preferably wherein the rear folded portion comprises a closed edge portion wherein the inner sheet of the rear folded portion is attached to the inner sheet of the elastic rear portion, wherein the closed edge portion extends from the rear waistline in the direction of the pocket opening.
Clause 7. The absorbent article according to any one of the preceding clauses, wherein the inner sheet comprises a spunbond nonwoven and/or meltblown nonwoven.
Clause 8. The absorbent article according to the preceding clause, wherein the inner sheet is a spunbond-meltblown-spunbond nonwoven.
Clause 9. The absorbent article according to clause 7 or 8, wherein the inner sheet has a basis weight of between 5-20gsm, preferably 6-17 gsm, more preferably 7-14 gsm, and most preferably 8-12 gsm.
Clause 10. The absorbent article according to any one of the preceding clauses, wherein the outer sheet comprises a spunbond and/or trough air bonded nonwoven.
Clause 11. The absorbent article according to the preceding clause, wherein the outer sheet is a spunbond nonwoven.
Clause 12. The absorbent article according to clause 10 or 11, wherein the outer sheet has a basis weight of between 8-25 gsm, preferably 9-20 gsm, more preferably 10-15 gsm.
Clause 13. The absorbent article according to any one of the preceding clauses, wherein the elastic front section comprises a front folded portion wherein the laminate of the elastic front section is folded from a front waistline of the elastic front section towards the topsheet of the absorbent body; and
   wherein the front folded portion extends from the front waistline to a free edge of the front folded portion to form a front guard pocket having a pocket opening defined between the free edge of the front folded portion and the topsheet of the absorbent body; wherein
   the front guard pocket comprises an hydrophilic inner and/or outer surface, preferably provided by a hydrophilic nonwoven, for example a hydrophilic nonwoven which forms the inner and/or outer sheet of the laminate.
Clause 14. The absorbent article according to the preceding clause, wherein the front folded portion comprises a first elastic member in proximity of and substantially parallel with the free edge, and a first attachment zone wherein the inner sheet and outer sheet are attached to each other wherein the first attachment zone is positioned in between the free edge and the first elastic member.
Clause 15. The absorbent article according to the preceding clause, wherein a maximum distance between the first elastic member and the free edge is less than 15mm, preferably less than 12mm, more preferably less than 9mm, and most preferably less than 6mm.
Clause 16. An absorbent article comprising an elastic front section, an elastic rear section, and an absorbent body extending in a longitudinal direction between the elastic front section and the elastic rear section,
   wherein the elastic front section and the elastic rear section each comprise a laminate consisting of an inner sheet, an outer sheet, and at least one elastic member positioned between the inner sheet and the outer sheet;
   wherein the absorbent body comprises a liquid pervious topsheet, a liquid impervious backsheet, and an absorbent core positioned between the topsheet and backsheet;
   wherein the elastic front section comprises a front folded portion wherein the laminate of the elastic front section is folded from a front waistline of the elastic front section towards the topsheet of the absorbent body; and
   wherein the front folded portion extends from the front waist line to a free edge of the front folded portion to form a front guard pocket having a pocket opening defined between the free edge of the front folded portion and the topsheet of the absorbent body;
   wherein
   the front guard pocket and/or the rear guard pocket comprise an hydrophilic inner and/or outer surface, preferably provided by a hydrophilic nonwoven, for example a hydrophilic nonwoven which forms the inner and/or outer sheet of the respective laminate.
Clause 17. The absorbent article according to clause 16, wherein the hydrophilic nonwoven is chosen from a treated nonwoven, such a treated nonwoven comprising polyethylene (PE), polypropylene (PP), polyester or polyethylene terephthalate (PET) or combinations thereof; or wherein the hydrophilic nonwoven is chosen from a nonwoven which substantially consist of a hydrophilic material(s); or wherein the hydrophilic nonwovens are chosen from nonwovens comprising a mixture of hydrophobic polymers blended or grafted with hydrophilic additives, preferably wherein the front folded portion comprises a first elastic member in proximity of and substantially parallel with the free edge, and a first attachment zone wherein the inner sheet and outer sheet are attached to each other wherein the first attachment zone is positioned in between the free edge and the first elastic member.
Clause 18. The absorbent article according to the preceding clause, wherein the hydrophilic nonwoven is chosen from treated nonwovens which have been treated with a surface treatment chosen from a surface coating, such as a surfactant coating, a plasma treatment, a corona discharge treatment, preferably a surface coating, preferably wherein a maximum distance between the first elastic member and the free edge is less than 15mm, preferably less than 12mm, more preferably less than 9mm, and most preferably less than 6mm.
Clause 19. The absorbent article according to any one of the preceding clauses 16-18, wherein the hydrophilic nonwoven is chosen from the group consisting of: a nonwoven which has been treated with a surfactant coating, a nonwoven which comprises a coating comprising hydrophilic agent, or nonwovens obtained by mixing a hydrophilic agent directly in a thermoplastic polymer resin before being processed into the nonwoven, e.g. via spunbonding, melt blowing, dry-laying, wet-laying, spunlacing or hydroentanglement, preferably wherein the front folded portion comprises a second attachment zone wherein the inner sheet and outer sheet are attached to each other, and wherein the second attachment zone is positioned in between the first elastic member and the front waistline.
Clause 20. The absorbent article according to the preceding clause, wherein the front folded portion comprises at least one second elastic member positioned between the first elastic member and the second attachment zone.
Clause 21. The absorbent article according to any one of the preceding clauses 16-20, wherein the front folded portion comprises a closed edge portion wherein the inner sheet of the front folded portion is attached to the inner sheet of the elastic front portion, wherein the closed edge portion extends from the front waistline in the direction of the pocket opening.
Clause 22. The absorbent article according to any one of the preceding clauses 16-21, wherein the inner sheet comprises a spunbond nonwoven and/or meltblown nonwoven.
Clause 23. The absorbent article according to the preceding clause wherein the inner sheet is a spunbond-meltblown-spunbond nonwoven.
Clause 24. The absorbent article according to clause 22 or 23, wherein the inner sheet has a basis weight of between 5-20 gsm, preferably 6-17 gsm, more preferably 7-14 gsm, and most preferably 8-12 gsm.
Clause 25. The absorbent article according to any one of the preceding clauses 16-24, wherein the outer sheet comprises a spunbond and/or trough air bonded nonwoven.
Clause 26. The absorbent article according to the preceding clause, wherein the outer sheet is a spunbond and/or through air bonded nonwoven.
Clause 27. The absorbent article according to clause 25 or 26, wherein the outer sheet has a basis weight of between 8-25 gsm, preferably 9-20 gsm, more preferably 10-15gsm.
Clause 28. The absorbent article according to any one of the preceding clauses, wherein the absorbent core comprises absorbent material and at least one channel, wherein less absorbent material per surface area is present in the at least one channel as compared to an area around the at least one channel, wherein preferably substantially no absorbent material is present in the at least one channel.
Clause 29. The absorbent article according to the preceding clause, wherein the at least one channel extends substantially in the longitudinal direction.
Clause 30. The absorbent article according to clause 28 or 29, wherein, at the at least one channel, a bottom core wrap of the absorbent core is attached to a top core wrap of the absorbent core.

With respect to the second aspect and related components, reference is made to the guard pocket(s) as described in WO 2024/141573 in the name of the applicant, which is incorporated herein by reference.

### BRIEF DESCRIPTION OF DRAWINGS

The invention will now be described in more details with respect to the drawings illustrating some preferred embodiments of the invention.
Figures 1 is a schematic top plan view of an exemplary embodiment of an absorbent article according to the first aspect of the invention, wherein the waist pocket is a rear waist pocket;
Figures 2 is a schematic top plan view of an alternative embodiment of an absorbent article according to the first aspect of the invention, wherein the waist pocket is a front waist pocket; and
Figures 3 is a schematic top plan view of a further alternative embodiment of an absorbent article according to the first aspect of the invention, wherein two waist pockets are provided, i.e. a rear waist pocket and a front waist pocket.

Following figures illustrate preferred embodiments of the second aspect of the invention which is defined in the clauses above, more in particular:
Figures 4A and 4B are schematic top plan views of an exemplary embodiment of an absorbent article according to the second aspect of the invention, wherein the elastic rear section is illustrated in its flat-out and folded state, respectively;
Figures 4C and 4D are schematic side views in the transversal direction of the elastic rear section of the exemplary embodiment of figures 4A and 4B, wherein the elastic rear section is illustrated in its flat-out and folded state, respectively;
Figures 5A and 5B are schematic top plan views of an exemplary embodiment of an absorbent article according to the second aspect of the invention, wherein the elastic front section is illustrated in its flat-out and folded state, respectively;
Figures 5C and 5D are schematic side views in the transversal direction of the elastic front section of the exemplary embodiment of figures 5A and 5B, wherein the front section is illustrated in its flat-out and folded state, respectively;
Figures 6A and 6B are schematic top plan views of an exemplary embodiment of an absorbent article according to the second aspect of the invention, wherein the elastic front and rear sections are illustrated in their flat-out and folded state, respectively;
Figure 7A is a partial top plan view of a preferred embodiment;
Figure 7B is a schematic side view of the preferred embodiment of figure 7A;
Figure 8A is a partial top plan view of an exemplary embodiment; and
Figures 8B and 8C are schematic side views of the exemplary embodiment of figure 8A.

### DETAILED DESCRIPTION

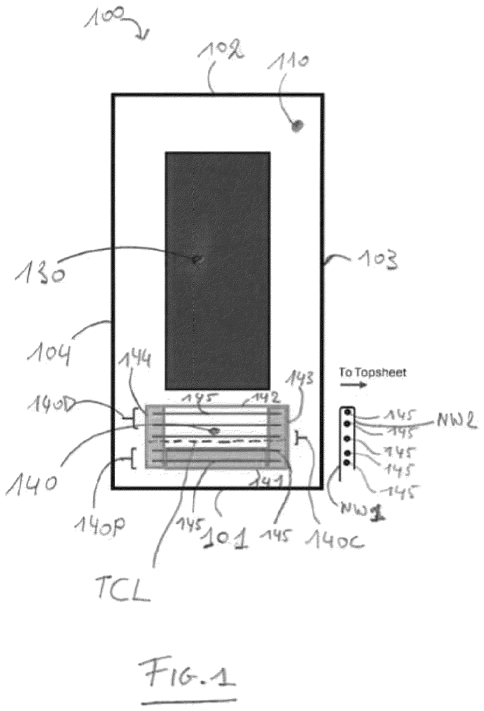

Figures 1, 2 and 3 illustrate mutually different embodiments of an absorbent article according to the first aspect of the invention.

More in particular, the figures illustrate an absorbent article 100 comprising a liquid pervious topsheet 110, a liquid impervious backsheet 120, and an absorbent core 130 which comprises absorbent material and which is positioned between the liquid pervious topsheet 110 and the liquid impervious backsheet 120; said absorbent article having a first and second longitudinal edge 103, 104 and a first and second transverse edge 101, 102; wherein the absorbent article comprises a waist pocket 140, 150 positioned near the first transverse edge 101, wherein the waist pocket 140, 150 comprises a proximal transversal portion 140P, 150P extending from a proximal transverse edge 141, 151 of the waist pocket 140, 150, which is proximal to the first transverse edge 101, towards a distal transversal edge of the waist pocket opposite to the proximal transverse edge; a distal transversal portion 140D, 150D extending from the distal transverse edge 142, 152 of the waist pocket towards the proximal transverse edge of the waist pocket; and a central transversal portion 140C, 150C between the proximal transversal portion and the distal transversal portion and comprising a transversal center line TCL, TCL' of the waist pocket; a first longitudinal side edge 143, 153, a second longitudinal side edge 144, 154, a first and second nonwoven layer NW1, NW2, NW1', NW2'.

The first and second nonwoven layer NW1, NW2, NW1', NW2' can be independently chosen from the hydrophilic nonwovens as described herein. Advantageously, improved pocket performance can thereby be achieved.

The figures 1 to 3 illustrate that the second nonwoven NW2 faces the topsheet, with other words the second nonwoven NW2 faces inwards.

The figures illustrate preferred embodiments wherein the waist pocket comprises one or more elastic members. However, it is clear for the skilled person that other embodiments wherein separate elastic members are not present in the waist pocket are also encompassed by the present application. The figures further illustrate at least one elastic material comprising elastic member 145, 155 positioned between the first and second nonwoven layer and extending substantially transversally between the first longitudinal side edge and the second longitudinal side edge, wherein the proximal transversal portion 140P, 150P is at least partially attached to a body facing side of the absorbent article 100 and the distal transversal portion 140D, 150D is at least partially unattached to the body facing side of the absorbent article, such that a waist pocket opening is formed between the distal transversal portion 140D, 150D and the topsheet 110; and wherein the at least one elastic member 145, 155 of the waist pocket is positioned such that a central grammage of elastic material in the central transversal portion 140C, 150C is lower than a distal grammage of elastic material in the distal transversal portion 140D, 150D. Beneficially, the at least one elastic member 145, 155 of the waist pocket 140, 150 is positioned such that the central grammage of elastic material in the central transversal portion 140C, 150C is lower than a proximal grammage of elastic material in the proximal transversal portion 140P, 150P. Further beneficially the distal transversal portion 140D, 150D comprises a first distal transversal sub-portion extending from the distal transverse edge of the waist pocket and a second distal transversal sub-portion between the first transversal sub-portion and the central transversal portion, and wherein a first distal grammage of elastic material in the first distal transversal sub-portion is higher than a second distal grammage of elastic material in the second distal transversal sub-portion. The proximal transversal portion 140P, 150P comprises a first proximal transversal sub-portion extending from the proximal transverse edge of the waist pocket and a second proximal transversal sub-portion between the first proximal transversal sub-portion and the central transversal portion, and wherein a first grammage of elastic material in the first proximal transversal sub-portion is higher than a second grammage of elastic material in the second proximal transversal sub-portion.

Each one of the proximal transversal portion 140P, 150P and the distal transversal portion 140D, 150D has a surface area which is substantially double of a surface area of the central transversal portion140C, 150C.

A surface area of each one of the first distal transversal sub-portion, second distal transversal sub-portion, first proximal transversal sub-portion, second proximal transversal sub-portion, and central transversal portion is substantially the same.

The absorbent article 100 comprises a pair of longitudinally extending leg cuffs, wherein the first longitudinal side edge and the second longitudinal side edge of the waist pocket are adhesively attached to the leg cuffs.

The proximal transverse edge 141, 151 of the waist pocket 140, 150 is adhesively attached to the topsheet. In the shown embodiments adhesive between the waist pocket 140, 150 and the topsheet are illustrated by the shaded parts of the waist pocket 140, 150.

The first and second nonwoven layer NW1, NW2, NW1', NW2' may consist of the same nonwoven material. Preferably, the first and second nonwoven layer NW1, NW2, NW1', NW2' consist of an at least partially folded nonwoven sheet comprising a folded connection. As illustrated, the at least partially folded nonwoven sheet is folded according to a C-fold or L-fold configuration. As indicated, a free portion of the at least partially folded nonwoven is facing the body facing side of the absorbent article 100, see the arrow ""to topsheet" in the figures. Preferably, and generally applicable, the free portion comprises at least a hydrophilic nonwoven, in particular a hydrophilic nonwoven as described herein. In this manner, containment of bodily exudate can be enhanced.

The at least one elastic member 145, 155 is adhesively attached between the first and second nonwoven layer NW1, NW2, NW1', NW2'. The first and/or second hydrophilic nonwoven layer NW1, NW2, NW1', NW2' comprises spunbond nonwoven, meltblown nonwoven, spunlaced nonwoven or combination thereof. Preferably the first and/or second nonwoven layer NW1, NW2, NW1', NW2' is a hydrophilic spunbond-meltblown-spunbond, SMS, nonwoven. The first and/or second hydrophilic nonwoven layer may have a grammage of between 4 - 15 gsm, preferably between 6 - 12 gsm, most preferably between 8 - 10 gsm.

The first and/or second hydrophilic nonwoven layer NW1, NW2, NW1', NW2'comprise, any one of polypropylene, polylactic acid, and biobased polyethylene which have been treated, in particular to induce a hydrophilic character. Preferably, the first and the second nonwoven layer NW1, NW2, NW1', NW2'consist of polypropylene which has been treated, in particular to induce a hydrophilic character. Generally, the treatment is preferably chosen from the treatments as described herein, such as the a surface treatment chosen from a surface coating, such as a surfactant coating, a plasma treatment, a corona discharge treatment, preferably a surface coating

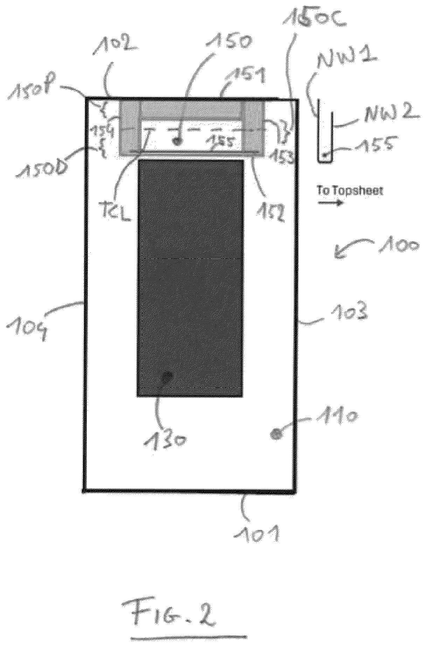

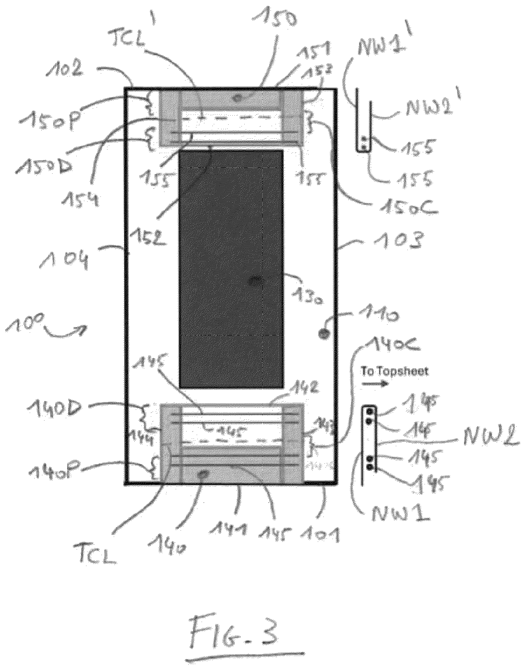

The at least one elastic member 145, 155 comprises a plurality of elastic members which are mutually positioned at a distance of between 4 and 10 mm of each other. In the embodiments of figures 2 and 3, the central transversal portion 140C, 150C is free of elastic members. In addition, in the embodiment of figures 2 and 3, the proximal transversal portion 150P is free of elastic members. In the illustrated embodiments the first transverse edge 101 is a rear transverse edge 101 of the absorbent article, and the waist pocket 140 is a rear waist pocket. When illustrated, the waist pocket 150 is a front waist pocket. The front waist pocket 150 can be provided in isolation as the embodiment of figure 2, or as a second waist pocket 150 in addition to a rear waist pocket 140 as the embodiment of figure 3. The at least one elastic member 145, 155 comprises at least one elastic strand. In the illustrated embodiments, an amount of elastic members 145, 155 in the central transversal portion 140C, 150C is lower than an amount of elastic members 145, 155 in the proximal transversal portion 140P, 150P. Further beneficially, an amount of elastic elements 145, 155 in the first distal transversal sub-portion is higher than an amount of elastic elements in the second distal transversal sub-portion. Alternatively or in addition an amount of elastic elements 145, 155 in the first proximal transversal sub-portion is higher than an amount of elastic elements in the second proximal transversal sub-portion.

In the illustrated embodiments the waist pocket 140, 150 is provided as a separate patch which is partially attached to the body facing side of the absorbent article and which is substantially rectangular shaped. The separate patch is preferably made from one or more hydrophilic nonwovens, that is the separate patch includes the first and second nonwoven as described herein.

Although it is not shown in the figures, it is preferred that the absorbent core if provided with at least one channel which is substantially free of absorbent material to improve liquid distribution and absorption.

It will be clear to the skilled person that in addition to the illustrated and described elastic members, additional elastic members may be present in the waist pocket. For example, an absorbent article as described above might have a total of between 1 and 6 elastic members in a front waist pocket and a total of between 2 and 10 elastic members in the rear waist pocket.

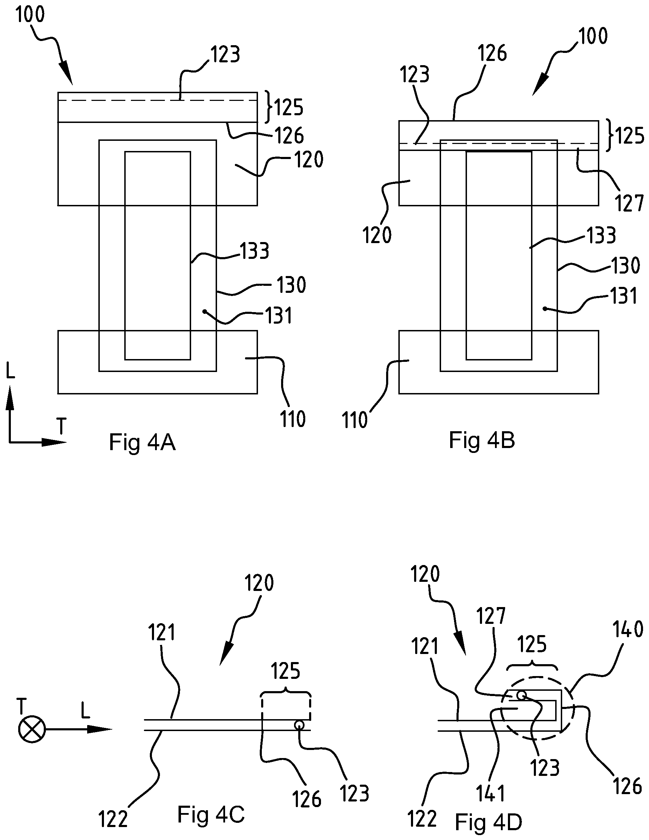

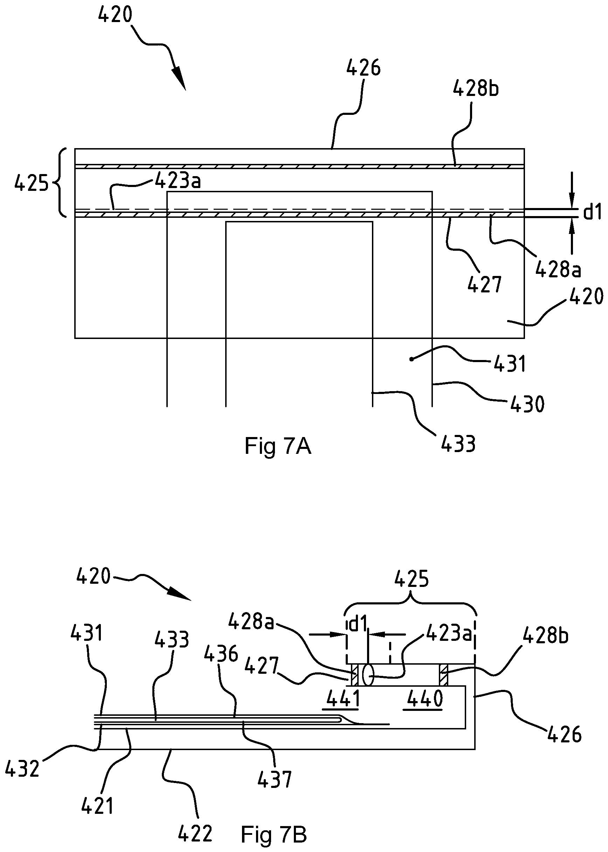

Figures 4A and 4B are schematic top views of an exemplary embodiment of an absorbent article 100 according to the second aspect of the invention. In figure 4A the elastic rear section 120 is shown in a flat-out state for illustrative purposes, and in figure 4B the elastic rear section 120 is shown in a folded state, which corresponds with the state of the absorbent article 100 when worn. Figures 4C and 4D are schematic side views in the transversal direction of the elastic rear section 120. Similarly figure 4C shows the elastic rear section 120 in the flat-out state for illustrative purposes, and figure 4D shows the elastic rear section 120 in the folded state. For reasons of clarity, figures 4C and 4D only show the elastic rear section 120. The absorbent body 130 which is partially supported by the elastic rear section 120 is not shown. A more complete view is presented in figure 7B. For the sake of completeness it is noted that elements in figures 4A and 4B are drawn as see-through elements. However, it will be clear to the skilled person that in figure 4A the absorbent body 130 which comprises the absorbent core 133 is supported by the front elastic section 110 and rear elastic section 120, and that in figure 4B the rear folded portion 125 extends partially over the absorbent body 130.

Figures 4A-4D illustrate an absorbent article 100, preferably baby pants, comprising an elastic front section 110, an elastic rear section 120, and an absorbent body 130 extending in the longitudinal direction L between the elastic front section 110 and the elastic rear section 120. The elastic rear section 120 comprises a laminate of an inner sheet 121 and an outer sheet 122. Between the inner sheet 121 and outer sheet 122 at least one elastic member 123 is provided. The absorbent body 130 comprises a liquid pervious topsheet 131, a liquid impervious backsheet 132, and an absorbent core 133 positioned between the topsheet 131 and backsheet 132. For reasons of clarity the backsheet 132 of the absorbent body 130 is not shown here. The elastic rear section 120 comprises a rear folded portion 125 where the laminate of the elastic rear section 120 is folded from a rear waistline 126 in the direction of the topsheet 131 of the absorbent body 130. In figures 4A and 4C the waistline 126 corresponds with an imaginary folding line at which the rear elastic section 120 is to be folded. In figures 4B and 4D, wherein the elastic rear section 120 is shown in its folded state, the waistline 126 is an actual waistline. The elastic member 123 is positioned within the rear folded portion 125 and is illustrated in a dashed line for illustrative purposes but it is clear to the skilled person that the elastic member 123 extends continuously in the transversal direction T. Additional elastic members may be provided in the rear folded portion 125 and/or in the remainder of the rear elastic section 120 other than the rear folded portion 125. This remainder is also referred to as rear unfolded portion and corresponds with the part of the elastic rear section 120 at the left-hand side of the indicated rear waistline 126 in figure 4C. The elastic member 123 and other elastic members that may be provided preferably are elastic strands which are at least partially covered with adhesive before being positioned within the laminate. The adhesive serves to keep the elastic members 123 in position and aids in keeping the laminate of the inner sheet 121 and outer sheet 122 together. The rear folded portion 125 extends from the rear waistline 126 to a free edge 127 of the rear folded portion 125 and thereby forms a rear guard pocket 140. The rear guard pocket has a pocket opening 141 defined between the free edge 127 of the rear folded portion 125 and the topsheet 131 of the absorbent body 130. The pocket opening 141 is situated close to an end portion of the absorbent core 133 and is configured for the intake of bodily exudates moving from the absorbent core 133 towards the rear waistline 126.

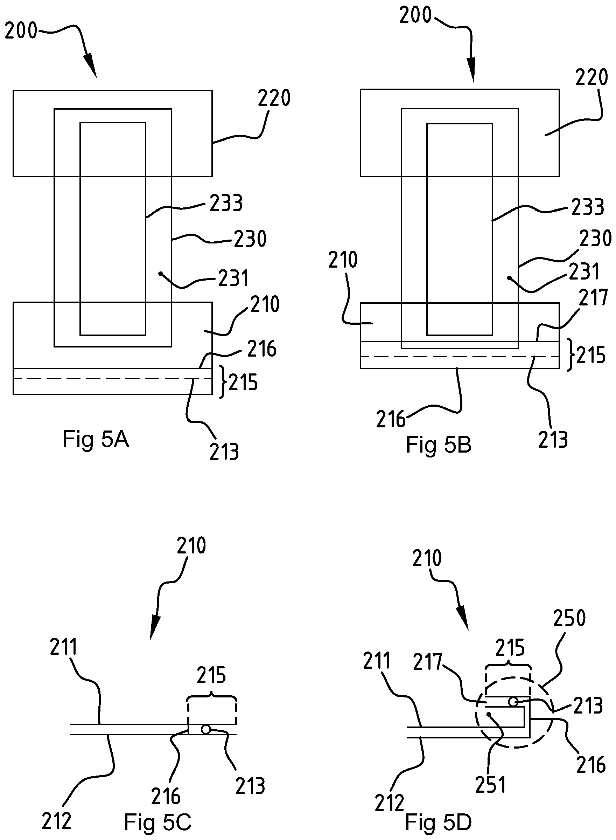

Figures 5A and 5B are schematic top views of an exemplary embodiment of an absorbent article 200 according to the second aspect of the invention. In figure 5A the elastic front section 210 is shown in a flat-out state for illustrative purposes, and in figure 5B the elastic front section 210 is shown in a folded state, which corresponds with the state of the absorbent article 200 when worn. Figures 5C and 5D are schematic side views in the transversal direction of the elastic front section 210. Similarly figure 5C shows the elastic front section 210 in the flat-out state for illustrative purposes, and figure 5D shows the elastic front section 210 in the folded state. For reasons of clarity, figures 5C and 5D only show the elastic front section 210. The absorbent body 230 which is partially supported by the elastic front section 210 is not shown. A more complete view is presented in figure 7B. For the sake of completeness it is noted that elements in figures 5A and 5B are drawn as see-through elements. However, it will be clear to the skilled person that in figure 5A the absorbent body 230 which comprises the absorbent core 233 is supported by the front elastic section 210 and rear elastic section 220, and that in figure 4B the front folded portion 215 extends partially over the absorbent body 230.

Figures 5A-5D illustrate an absorbent article 200, preferably baby pants, comprising an elastic front section 210, an elastic rear section 220, and an absorbent body 230 extending in the longitudinal direction L between the elastic front section 210 and the elastic rear section 220. The elastic front section 210 comprises a laminate of an inner sheet 211 and an outer sheet 212. Between the inner sheet 211 and outer sheet 212 at least one elastic member 213 is provided. The absorbent body 230 comprises a liquid pervious topsheet 231, a liquid impervious backsheet 232, and an absorbent core 233 positioned between the topsheet 231 and backsheet 232. For reasons of clarity the backsheet 232 of the absorbent body 230 is not shown here. The elastic front section 210 comprises a front folded portion 215 where the laminate of the elastic front section 210 is folded from a front waistline 216 in the direction of the topsheet 231 of the absorbent body 230. In figures 5A and 5C the waistline 216 corresponds with an imaginary folding line at which the front elastic section 210 is to be folded. In figures 5B and 5D, wherein the elastic front section 210 is shown in its folded state, the waistline 216 is an actual waistline. The elastic member 213 is positioned within the front folded portion 215 and is illustrated in a dashed line for illustrative purposes but it is clear to the skilled person that the elastic member 213 extends continuously in the transversal direction T. Additional elastic members may be provided in the front folded portion 215 and/or in the remainder of the front elastic section 210 other than the front folded portion 215. This remainder is also referred to as front unfolded portion and corresponds with the part of the elastic front section 210 at the left-hand side of the indicated front waistline 216 in figure 5C. The elastic member 213 and other elastic members that may be provided preferably are elastic strands which are at least partially covered with adhesive before being positioned within the laminate. The adhesive serves to keep the elastic members 213 in position and aids in keeping the laminate of the inner sheet 221 and outer sheet 222 together. The front folded portion 215 extends from the front waistline 216 to a free edge 217 of the front folded portion 215 and thereby forms a front guard pocket 250. The front guard pocket 250 has a pocket opening 251 defined between the free edge 217 of the front folded portion 215 and the topsheet 231 of the absorbent body 230. The pocket opening 251 is situated close to an end portion of the absorbent core 233 and is configured for the intake of bodily exudates moving from the absorbent core 233 towards the front waistline 216.

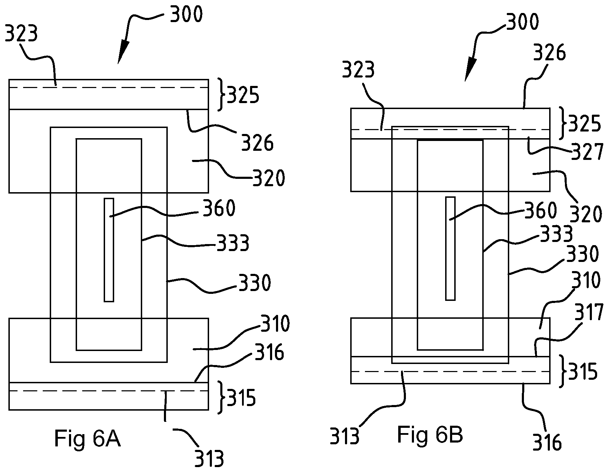

Figures 6A and 6B are schematic top views of an exemplary embodiment of an absorbent article 300 according to the second aspect of the invention. In figure 6A the elastic front section 310 and the elastic rear section 320 are shown in a flat-out state for illustrative purposes, and in figure 6B the elastic front section 310 and the elastic rear section 320 are shown in a folded state, which corresponds with the state of the absorbent article 300 when worn. For the elastic rear section 320 a similar description as given in view of elastic rear section 120 in figures 4A-4D applies, mutatis mutandis. A further detailed description is therefore omitted here for reasons of conciseness. For the elastic front section 310 a similar description as given in view of elastic front section 210 in figures 5A-5D applies, mutatis mutandis. A further detailed description is therefore omitted here for reasons of conciseness. In essence, the embodiment of figures 6A and 6B corresponds with an absorbent article 300, preferably baby pants, which combines the elastic rear section 120, 320 of the embodiment of figures 4A-4D, with the elastic front section 210, 310 of the embodiment of figures 5A-5D, with the additional feature that the absorbent core 333 is provided with a channel 360. In the channel 360, or multiple channels 360 (not shown) less absorbent material per surface area is present as compared to an area of the absorbent core 333 around the channel 360. Preferably substantially no absorbent material is present in the at least one channel 360. It is clear that the provision of one or more channels 360 is not necessarily and exclusively linked to the embodiment of figures 6A-6B, and that one or more channels 360 may be provided in the embodiments of figures 4A-4D and figures 5A-5D as well. For the sake of conciseness a further detailed description of possible absorbent core configurations with one or more channels 360 are omitted here. Absorbent articles comprising an absorbent core with one or more channels between a topsheet and a backsheet are well known. Examples of such absorbent articles are described for example in patents EP 3 403 630 B1, EP 3 403 632 B1 and patent application EP 3 403 631 A1 in the name of the applicant, which are incorporated herein by reference. The applicant has found that it is especially beneficial in embodiments of absorbent articles with elastic front and/or rear sections with corresponding front and/or rear guard pockets to have an absorbent core with one or more channels wherein the majority of the channel(s) extends substantially in the longitudinal direction, i.e. towards the front and/or rear guard pocket.

Figure 7A is a top plan view of an elastic rear section 420 of an absorbent article according to a preferred embodiment. Figure 7B is a schematic side view of the elastic rear section 420 as illustrated in figure 7A. Figures 7A and 7B show an elastic rear section 420 comprising a laminate of an inner sheet 421, an outer sheet 422, and at least one elastic member 423 positioned between the inner sheet 421 and the outer sheet 422. The elastic rear section 420 comprises a rear folded portion 425 wherein the laminate is folded from a rear waistline 426 of the elastic rear section towards the topsheet 431 of the absorbent body 430. The rear folded portion 425 extends from the rear waistline 426 to a free edge 427 of the rear folded portion 425 to form a rear guard pocket 440 having a pocket opening 441 defined between the free edge 427 of the rear folded portion 425 and the topsheet 431 of the absorbent body 430. The rear folded portion 425 comprises a first elastic member 426A in proximity of and substantially parallel with the free edge 427. The maximum distance d1 between the first elastic member 426A and the free edge 427 should be as small as possible and less than 15mm. Preferably the distance d1 is less than 12mm, more preferably less than 9mm, and most preferably less than 6mm. In close proximity of the first elastic member 426A, and preferably adjacent thereto, there is provided a first attachment zone 128a, in the form of a strip of adhesive, wherein the inner sheet 421 and outer sheet 422 are attached to each other. The first attachment zone 428a is positioned between the free edge 427 and the first elastic member 426A. In the illustrated embodiment the rear folded portion 425 comprises a second attachment zone 428b wherein the inner sheet 421 and outer sheet 422 are attached to each other. The second attachment zone 128b is positioned in between the first elastic member 426A and a rear baseline of the rear guard pocket. The baseline corresponds with the position from whereon the rear folded portion 425 starts to open up towards the free edge 427, i.e. from where there is a distance between the inner sheet 421 of the rear folded portion 425 and the inner sheet 421 of the unfolded portion of the elastic rear section 420. In the illustrated embodiment the baseline of the rear guard pocket is the rear waistline 426. In a further embodiment the rear folded portion 425 may comprise at least one second elastic member (not shown) positioned between the first elastic member 426A and the second attachment zone 428b, preferably in close proximity of the second attachment zone 428b. On the other hand, it is clear that other embodiment exist wherein the second attachment zone 428b is not provided. Figure 7B further illustrates that the absorbent body 430 is provided partially on top of the inner sheet 421 of the elastic rear section 420. The absorbent body 430 comprises a liquid pervious topsheet 431, a liquid impervious backsheet 432, and an absorbent core 433 positioned between the topsheet 431 and backsheet 432. The absorbent core 433 comprises a bottom core wrap 437, a top core wrap 436 and absorbent material therebetween.

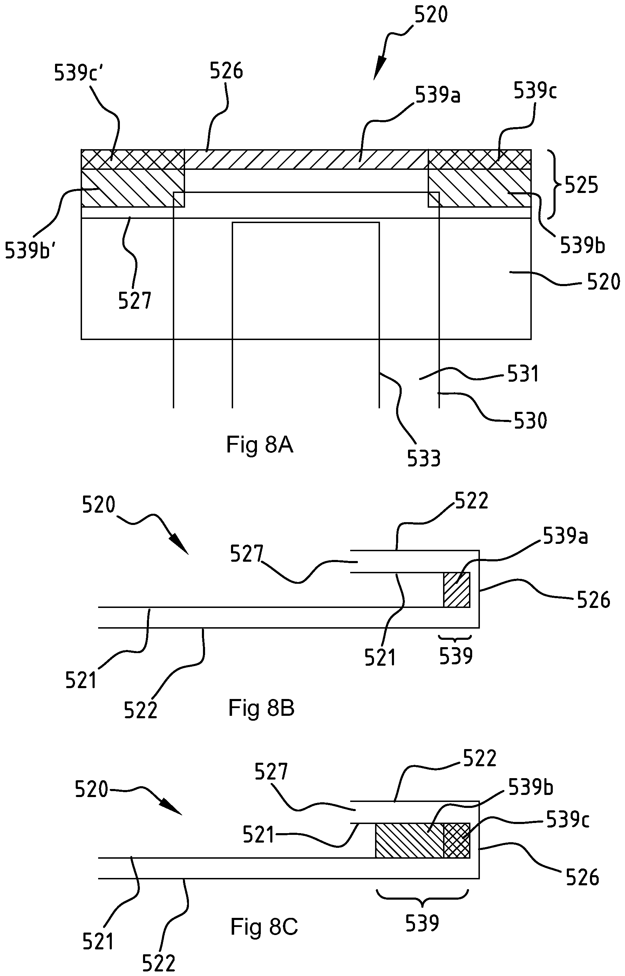

Figure 8A is a top plan view of an elastic rear section 520 of an absorbent article according to a preferred embodiment. Figures 8B and 8C are schematic side views of the elastic rear section 520 as illustrated in figure 8A. More in particular figure 8B shows a side view of a cross section taken along a longitudinal center line, and figure 8C shows a side view of a cross section taken along a longitudinal side line. It is noted that figures 8A, 8B and 8C are intended to illustrate an embodiment of a rear elastic section 520 having a closed edge portion 539, and therefore these figures do not show any elastic members or attachment zones in the laminate of the elastic rear section 520 for the sake of clarity. At the closed edge portion 539 the inner sheet 521 of the rear folded portion 525 is attached to or connected with the inner sheet 521 of the unfolded portion of the elastic rear section. The closed edge portion 539 provides additional stability to the guard pocket formed by the rear folded portion 525, because the rear folded portion 525 is partially adhered to unfolded portion. The closed edge portion 539 is configured such that there is still a sufficiently large part of the rear folded portion 525 that opens up in an adequate manner to receive bodily exudates. More in particular, the illustrated closed edge portion 539 comprises three edge subportions 539a, 539b and 539b'. Subportion 539a is substantially strip shaped and extends parallel to the rear waistline 526 and preferably adjacent thereto. Subportions 539b and 539b' are substantially rectangular and are positioned at each corner of the elastic rear section 520 which borders the rear waistline. Subportions 539a and subportions 539b, 539b' partially overlap in overlap sections 539c, 539c'. The closed edge portion 539 is formed by applying adhesive on the inner sheet 521 in accordance with the to be formed closed edge portion 539 prior to folding the elastic rear section 520. In the illustrated embodiment the baseline of the rear guard pocket is determined by the closed edge portion 539, especially by subportion 539a. It is clear that each of the indicated subportions 539a, 539b, 539b' can be provided separately, or in any combination.

Although figures 7A-7B and 8A-8C and the corresponding description is aimed at specific embodiments of a rear elastic section, the illustrated and described features apply in a similar manner to embodiments of a front elastic section, mutatis mutandis. Front and rear elastic sections or panels of absorbent articles such as baby diapers or baby pants are often made from similar materials but might have slightly different dimensions and configurations. For example, a rear elastic section according to an embodiment of the invention might have a width (transversal direction) of about 405 mm and a length (longitudinal direction) of about 170 mm in the folded state, wherein the rear folded portion has a length of about 60 mm, while a front elastic section according to an embodiment of the invention might have a width (transversal direction) of about 405 mm and a length (longitudinal direction) of about 130 mm in the folded state, wherein the front folded portion has a length of about 55 mm. It is clear that these dimensions may vary depending on the size of the absorbent article.

It will be clear to the skilled person that in addition to the illustrated and described elastic members, additional elastic members may be present in the elastic front and/or rear sections, in the folded and/or unfolded portions. For example, an absorbent article having the exemplary dimensions as described above might have a total of between 1 and 6 elastic members in the front or rear folded portions and a total of between 8 and 24 elastic members in the front or rear unfolded portions.

### Glossary

As used in the present application, the following terms have the following meanings:
"A", "an", and "the" as used herein refers to both singular and plural referents unless the context clearly dictates otherwise. By way of example, "an edge barrier" refers to one or more than one edge barrier.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or variations are appropriate to perform in the disclosed invention. However, it is to be understood that the value to which the modifier "about" refers is itself also specifically disclosed.

"Absorbent article", "absorbent garment", "absorbent product", "absorbing article", "absorbing garment", "absorbing product" and the like as used herein are used interchangeably and refer to devices that absorb and contain bodily exudates, and more specifically, refers to devices that are placed against or in proximity to the body of the wearer to absorb and contain the various liquids discharged from the body. Absorbent articles include but are not limited to feminine hygiene garments, baby diapers and pants, adult incontinence garments, various diaper and pants holders, liners, towels, absorbent inserts and the like.

"Absorbent core" as used herein refers to a three-dimensional part of the absorbent structure, comprising liquid-absorbing material, useful to permanently absorb and/or retain bodily exudates. "Absorbent component" as used herein refers to a structural constituent of an absorbent article, e.g., a piece of an absorbent core, such as one of multiple pieces in a multi-piece absorbent core. "Absorbent element" as used herein refers to a part of a functional constituent of an absorbent structure, e.g., an acquisition layer, a dispersion layer, core layer or a release structure formed of a material or materials having particular liquid handling characteristics suitable for the specific function.

"Absorbent fibrous polymer material" as used herein refers to an absorbent polymer material which is in threadlike from such as fibers, filaments, and the like so as to be less flowable in the dry state than particulates.

"Absorbent insert" as used herein refers to a device adapted for insertion into an "Absorbent layer" as used herein refers to a term referring to a discrete, identifiable sheet-like or web-like element of an absorbent article which may remain detached and relatively movable with respect to another such element or may be attached or joined so as to remain permanently associated with another such element. Each absorbent layer may itself include a laminate or combination of several layers, sheets and/or webs of similar or diverse compositions.

"Absorbent polymer material", "absorbent gelling material", "AGM", "superabsorbent", "superabsorbent material", "superabsorbent polymer", "SAP" and the like as used herein are used interchangeably and refer to any suitable particulate (e.g., flaked, particulate, granular, or powdered) or fibrous cross linked polymeric materials that can absorb at least 5 times and preferably at least about 10 times or more its weight of an aqueous 0.9% saline solution as measured using the Centrifuge Retention Capacity test (EDANA 441.2-01).

"Absorbent polymer material area" as used herein refers to the area of the absorbent structure wherein adjacent layers are separated by a multiplicity of absorbent polymer material. Incidental contact areas between these adjacent layers within the absorbent particulate polymer material area may be intentional (e.g bond area's) or unintentional (e.g. manufacturing artifacts).

"Absorbent particulate polymer material" as used herein refers to an absorbent polymer material which is in particulate form such as powders, granules, flakes and the like so as to be flowable in the dry state.

"Absorption" as used herein refers to the process by which a liquid is taken up within a material. "Absorption rate" as used herein refers to the rate of absorption of liquid, i.e. the amount of liquid which is absorbed per unit of time, typically by an absorbent component, element and/or absorbent layer of the absorbent article, structure and/or core.

"Acquisition layer", "acquisition region", "acquisition surface" or "acquisition material" and the like as used herein refer to the layer overlying the absorbent core having a faster liquid uptake and/or distribution capability.

"Absorbency" is the ability of a material to take up fluids by various means including capillary, osmotic, solvent, chemical and/or other action.

"Adult incontinence garment" as used herein refers to absorbent articles intended to be worn by incontinent adults, for absorbing and containing bodily exudates.

"Adhesion" as used herein refers to the force that holds different materials together at their interface.

"Adhesive" as used herein refers to a material, which may or may not be flowable in solution or when heated, that is used to bond materials together.

"Adsorption" as used herein refers to the process by which a liquid is taken up by the surface of a material.

"Airlaying" as used herein refers to forming a web by dispersing fibers or particles in an air stream and condensing them from the air stream onto a moving screen by means of a pressure and/or vacuum; a web of fibers produced by airlaying is herein referred to an "airlaid"; an airlaid web bonded by one or more techniques to provide fabric integrity is herein referred to an "airlaid nonwoven".

"Apparent density", "density" as used herein refers to the basis weight of the sample divided by the caliper with appropriate unit conversions incorporated therein. Apparent density used herein has the unit g/cm3.

"Attach", "attached" and "attachment" as used herein are synonymous with their counterparts of the terms "fasten", "affix", "secure", "bind", "join" and "link".

"Baby diaper" as used herein refers to absorbent articles intended to be worn by children, for absorbing and containing bodily exudates which the user draws up between the legs and fastens about the waist of the wearer.

"Baby pants" as used herein refers to absorbent articles marketed for use in transitioning children from diapers to underwear intended to cover the lower torso of children, so as to absorb and contain body exudates which article is generally configured like a panty garment and manufactured with a completed waist encircling portion, thereby eliminating the need for the user to fasten the article about the waist of the wearer.

"Back region" as used herein refers to the portion of an absorbent article or part thereof that is intended to be positioned proximate the back of a wearer.

"Backing" as used herein refers to a web or other material that supports and reinforces the back of a product.

"Basis weight" is the weight per unit area of a sample reported in grams per square meter, g/m2 or gsm.

"Bodily exudates", "body exudates", "bodily fluids", "body fluids", "bodily discharges", "body discharges", "fluid(s)", " liquid(s)", "fluid(s) and liquid(s) and the like as used herein are used interchangeably and refer to, but are not limited to urine, blood, vaginal discharges, breast milk, sweats and fecal matter.

"Binder", "adhesive", "glue", "resins", "plastics" and the like as used herein are used interchangeably and refer to substances, generally in a solid form (e.g. powder, film, fiber) or as a foam, or in a liquid form (e .g. emulsion, dispersion, solution) used for example by way of impregnation, spraying, printing, foam application and the like used for attaching or bonding functional and/or structural components, elements and materials, for example including heat and/or pressure sensitive adhesives, hot-melts, heat activated adhesives, thermoplastic materials, chemical activated adhesives/solvents, curable materials and the like.

"Bond strength" as used herein refers to the amount of adhesion between bonded surfaces. It is a measure of the stress required to separate a layer of material from the base to which it is bonded. "Capillary action", "capillarity", or "capillary motion" and the like as used herein are used to refer to the phenomena of the flow of liquid through porous media.

"Chassis" as used herein refers to a foundational constituent of an absorbent article upon which the remainder of the structure of the article is built up or overlaid, e.g., in a diaper, the structural elements that give the diaper the form of briefs or pants when configured for wearing, such as a backsheet, a topsheet, or a combination of a topsheet and a backsheet.

"Cellulose fibers" as used herein refers to naturally occurring fibers based on cellulose, such as, for example cotton, linen, etc.; wood pulp fibers are one example of cellulose fibers; man-made fibers derived from cellulose, such as regenerated cellulose (rayon), or partially or fully acetylated cellulose derivatives (e.g. cellulose acetate or triacetate) are also considered as cellulose fibers. "Cluster" or the like as used herein refers to an agglomeration of particles and/or fibers. "Chemically stiffened fibers", chemically modified fibers", "chemically cross-linked fibers", "curly fibers" and the like as used herein are used interchangeably and refer to any fibers which have been stiffened by chemical means to increase stiffness of the fibers under both dry and aqueous conditions, for example by way of addition of chemical stiffening agents (e.g. by coating, impregnating, etc.), altering the chemical structure of the fibers themselves (e.g. by cross-linking polymer chains, etc.) and the like.

"Cohesion" as used herein refers to the resistance of similar materials to be separated from each other.

"Compartment" as used herein refers to chambers, cavities, pockets and the like.

"Comprise," "comprising," and "comprises" and "comprised of" as used herein are synonymous with "include", "including", "includes" or "contain", "containing", "contains" and are inclusive or open-ended terms that specify the presence of what follows e.g. a component and do not exclude or preclude the presence of additional, non-recited components, features, elements, members, steps, known in the art or disclosed therein.

"Coverstock" as used herein refers to a lightweight non-woven material used to contain and conceal an underlying absorbent core material; examples are the facing layer or materials that cover the absorbent cores of feminine hygiene garment s, baby diapers and pants and adult incontinence garments.

"Crotch region" of an absorbent article as used herein refers to about 50% of the absorbent article's total length (i.e., in the y-dimension), where the crotch point is located in the longitudinal center of the crotch region. That is, the crotch region is determined by first locating the crotch point of the absorbent article, and then measuring forward and backward a distance of 25% of the absorbent article's total length.

"Cross direction (CD)", "lateral" or "transverse" and the like as used herein are used interchangeably and refer to a direction which is orthogonal to the longitudinal direction and includes directions within ±45° of the transversal direction.

"Curing" as used herein refers to a process by which resins, binders or plastics are set into or onto fabrics, usually by heating, to cause them to stay in place; the setting may occur by removing solvent or by cross-linking so as to make them in soluble.

"Diaper", "conventional diaper", "diaper-like", "diaper-like garment" and the like as used herein are used interchangeably and refer to disposable absorbent articles, which typically include a front waist portion and a back waist portion which may be releasable connected about the hips of the wearer during use by conventional fasteners such as adhesive tape fasteners or hook and loop type fasteners. In use, the article is positioned between the legs of the wearer and the fasteners are releasable attached to secure the back waist portion to the front waist portion of the diaper, thereby securing the diaper about the waist of the wearer. The front waist portion and a back waist portion are connected by relatively non-stretchable or stretchable members (the term "stretchable" as used herein refers to materials that are extensible when forces are applied to the material, and offer some resistance to extension). Hence, such articles are generally not configured to be pulled up or down over the hips of the wearer when the fasteners are attached.

"Dispersion layer", "dispersion region", "dispersion surface" or "dispersion material" and the like as used herein refer to the layer overlying the absorbent core having a faster liquid uptake and dispersion capability.

"Disposable" is used herein to describe articles that are generally not intended to be laundered or otherwise restored or reused (i.e., they are intended to be discarded after a single use and, preferably, to be recycled, composted or otherwise disposed of in an environmentally compatible manner).

"Drylaying" as used herein refers to a process for making a nonwoven web from dry fiber; these terms apply to the formation of carded webs, as well as to the air laying formation of random webs; a web of fibers produced by drylaying is herein referred to as a "drylaid"; a drylaid web bonded by one or more techniques to provide fabric integrity is herein referred to a "drylaid nonwoven".

"Dry strength" as used herein refers to the strength of a joint determined in dry state conditions, immediately after drying under specified conditions or after a period of conditioning in the standard laboratory atmosphere.

"Essentially cellulose free", "substantially fluffless" or "little to no cellulose fibers" as used herein refers to an absorbent article, structure, core component and/or element containing less than 20% by weight cellulosic fibers, less than 10% cellulosic fibers, less than 5% cellulosic fibers, no cellulosic fibers, or no more than an immaterial amount of cellulosic fibers which do not materially affect the thinness, flexibility or absorbency thereof.

"Essentially fluffless" or "little to no fluff pulp" as used herein refers to an absorbent article, structure, core, component and/or element containing less than 20% by weight fluff pulp, less than 10% fluff pulp, less than 5% fluff pulp, no fluff pulp, or no more than an immaterial amount of fluff pulp which do not materially affect the thinness, flexibility or absorbency thereof.

"Fabric" as used herein refers to a sheet structure made from fibers, filaments and/or yarns. "Feminine hygiene garments" as used herein refer to absorbent hygiene articles intended to be worn by woman, for absorbing and containing body exudates.

"Fiber" as used herein refers to the basic threadlike structure from which nonwovens, yarns and textiles are made. It differs from a particle by having a length at least 4 times its width; "Natural fibers" are either of animal (wool, silk), vegetable (cotton, flax, jute) or mineral (asbestos) origin, while "Man-made fibers" may be either polymers synthesized from chemical compounds (polyester, polypropylene, nylon, acrylic etc.) or modified natural polymers (rayon, acetate) or mineral (glass). "Fiber" and "filament" are used interchangeably.

"Fluff pulp" or "Pulp fluff" as used herein refers to wood pulp specially prepared to be drylaid. The fibers can be either natural or synthetic or a combination thereof.

"Front region" as used herein refers to the portion of an absorbent article or part thereof that is intended to be positioned proximate the front of a wearer.

"Garment facing layer" as used herein refers to elements of the chassis that form the outer surface of the absorbent article, such as the backsheet, the side panels, the waist fasteners, and the like, when such elements are present.

"Heat activated adhesive" as used herein refers to a dry adhesive that is rendered tacky or fluid by application of heat or heat and pressure to the assembly.

"Heat sealing adhesive" as used herein refers to a thermoplastic adhesive which is melted between the adherent surfaces by heat application to one or both of the adjacent adherent surfaces.

"High loft" as used herein refers to general term of low density, thick or bulky fabrics.

"Hot-melt adhesive" as used herein refers to a solid material that melts quickly upon heating, then sets to a firm bond upon cooling; used for almost instantaneous bonding.

"Hydrophilic" as used herein refers to having an affinity for being wetted by water or for absorbing water.

"Hydrophobic" as used herein refers to lacking the affinity for being wetted by water or for absorbing water.

"Immobilization layer" as used herein refers to a layer able to be applied to the absorbent polymer material or absorbent polymer material area with the intent to gather, bond and/or immobilize absorbent material and/or absorbent layer.

"Join", "joined" and "joining" as used herein refers to encompassing configurations wherein an element is directly secured to another element by affixing the element directly to the other element, as well as configurations wherein the element is indirectly secured to the other element by affixing the element to an intermediate member or members which in turn is or are affixed to the other element.

"Knitting" as used herein refers to the technique for interlocking loops of fibers with needles or similar devices.

"Layer" refers to identifiable components of the absorbent article, and any part referred to as a "layer" may actually comprise a laminate or combination of several sheets or webs of the requisite type of materials. As used herein, the term "layer" includes the terms "layers" and "layered." "Upper" refers to the layer of the absorbent article which is nearest to and/ or faces the wearer facing layer; conversely, the term "lower" refers to the layer of the absorbent article which is nearest to and/or faces the garment facing layer. "Layer" is three dimensional structure with a x dimension width, y dimension length, and z-dimensions thickness or caliper, said x-y dimensions being substantially in the plane of the article, however it should be noted that the various members, layers, and structures of absorbent articles according to the present invention may or may not be generally planar in nature, and may be shaped or profiled in any desired configuration .

"Machine direction (MD)", "longitudinal" and the like as used herein are used interchangeably and refer to a direction running parallel to the maximum linear dimension of the structure and includes directions within ±45° of the longitudinal direction.

"Major surface" as used herein refers to a term used to describe the surfaces of greatest extent of a generally planar or sheet-like structural element and to distinguish these surfaces from the minor surfaces of the end edges and the side edges, i.e., in an element having a length, a width, and a thickness, the thickness being the smallest of the three dimensions, the major surfaces are those defined by the length and the width and thus having the greatest extent.

"Mass flow" as used herein refers to the flow of a liquid from one absorbent element or component to another absorbent element or component by channel flow action.

"Mechanical bonding" as used herein refers to a method of bonding fibers by entangling them. This can be achieved by needling, stitching with fibers or by the use of high-pressure air or water jets and the like.

"Nonwoven" as used herein refers to manufactured sheet, web or batt of directionally or randomly orientated fibers, bonded by friction, and/or cohesion and/or adhesion, excluding paper and products which are woven, knitted, tufted, stitch-bonded incorporating binding yarns or filaments, or felted by wet-milling, whether or not additionally needled. The fibers may be of natural or man-made origin and may be staple or continuous filaments or be formed in situ. Commercially available fibers have diameters ranging from less than about 0.001 mm to more than about 0.2 mm and they come in several different forms: short fibers (known as staple, or chopped), continuous single fibers (filaments or monofilaments), untwisted bundles of continuous filaments (tow), and twisted bundles of continuous filaments (yarn). Nonwoven fabrics can be formed by many processes such as melt blowing, spun bonding, solvent spinning, electrospinning, and carding. The basis weight of nonwoven fabrics is usually expressed in grams per square meter (gsm).

"Pant", "training pant", "closed diapers", "prefastened diapers", "pull-on diapers" and "diaper-pants" and the like as used herein are used interchangeably and refer to absorbent articles which are typically applied to the wearer by first leading the feet into the respective leg openings and subsequently pulling the pants from the feet to waist area over the hips and buttocks of the wearer and which are capable of being pulled up or down over the hips of the wearer. Typically, such articles may include a front waist portion and a back waist portion which may be connected about the hips of the wearer by integral or releasable members. A pant may be preformed by any suitable technique including, but not limited to, joining together portions of the article using refastenable and/or non-refastenable bonds (e.g., seam, weld, adhesive, cohesive bond, fastener, etc.). A pant may be preformed anywhere along the circumference of the article (e.g., side fastened, front waist fastened).

"Polymer" as used herein refers to but is not limited to, homopolymers, copolymers, such as for example, block, graft, random and alternating copolymers, terpolymers, etc. and blends and modifications thereof. Unless otherwise specifically limited, the term "polymer" includes all possible spatial configurations of the molecule and include, but are not limited to isotactic, syndiotactic and random symmetries.

"Rear" as used herein refers to the portion of an absorbent article or part thereof that is intended to be positioned proximate the back of the wearer.

"Release structure", "release region", "release surface" or "release material" and the like as used herein are used interchangeably and refer to a structure in fluid communication with the absorbent core having a larger relative liquid absorption capacity and/or rate allowing it to quickly take up, temporarily hold and releasing liquids.

"Resin" as used herein refers to a solid or semisolid polymeric material.

"Thermobonding" as used herein refers to a method of bonding fibers by the use of heat and/or high-pressure.

"Thermoplastic" as used herein refers to polymeric materials that have a melting temperature and can flow or be formed into desired shapes on the application of heat at or below the melting point. "Ultrasonic" as used herein refers to the use of high frequency sound to generate localized heat through vibration thereby causing thermoplastic fibers to bond to one another.

"Water-absorbing", "liquid-absorbing", "absorbent", "absorbing" and the like as used herein are used interchangeably and refer to compounds, materials, products that absorb at least water, but typically also other aqueous fluids and typically other parts of bodily exudates such as at least urine or blood.

"Wearer facing layer" as used herein refers to elements of the chassis that form the inner surface of the absorbent article, such as the topsheet, the leg cuffs, and the side panels, etc., when such elements are present.

"Weaving" as used herein refers to the process of interlacing two or more sets of yarns at right angles to form a fabric; a web of fibers produced by weaving is herein referred to as a "woven". "Web material" as used herein refers to an essentially endless material in one direction, i.e. the longitudinal extension or the length, or the x- direction in Cartesian coordinates relative to the web material. Included in this term is an essentially unlimited sequence of pieces cut or otherwise separated from an essentially endless material. Often, though not necessarily, the web materials will have a thickness dimension (i.e. the z-direction) which is significantly smaller than the longitudinal extension (i.e. in x-direction). Typically, the width of web materials (they-direction) will be significantly larger than the thickness, but less than the length. Often, though not necessarily, the thickness and the width of such materials is essentially constant along the length of the web. Without intending any limitation, such web materials may be cellulosic fiber materials, tissues, woven or nonwoven materials and the like. Typically, though not necessarily, web materials are supplied in roll form, or on spools, or in a folded state in boxes. The individual deliveries may then be spliced together to form the essentially endless structure. A web material may be composed of several web materials, such as multilayer non-woven, coated tissues, nonwoven/film laminates. Web materials may comprise other materials, such as added binding material, particles, hydrophilizing agents and the like.

"Wet burst strength" is a measure of a layer's ability to absorb energy, when wet and subjected to deformation normal to the plane of the web.

"Wet strength" as used herein refers to the strength of a joint determined immediately after removal from a liquid in which it has been immersed under specified conditions of time, temperature and pressure. The term is commonly used in the art to designate strength after immersion in water. "Wetlaying" as used herein refers to the forming a web from an aqueous dispersion of fibers by applying modified paper making techniques; a web of fibers produced by wetlaying is herein referred to as a "wetlaid".

"Wood pulp" as used herein refers to cellulosic fibers used to make viscose rayon, paper and the absorbent cores of products such as feminine hygiene garments, baby diapers and pants and adult incontinence garments.

"X-y dimension" as used herein refers to the plane orthogonal to the thickness of the article, structure or element. The x- and y-dimensions correspond generally to the width and length, respectively, of the article, structure or element.

"Z-dimension" as used herein refers to the dimension orthogonal to the length and width of the article, structure or element. The z-dimension corresponds generally to the thickness of the article, structure or element.

The skilled person will appreciate on the basis of the above description that the invention can be embodied in different ways and on the basis of different principles. The invention is not limited to the above described embodiments. The above described embodiments and the figures are purely illustrative and serve only to increase understanding of the invention. The invention will not therefore be limited to the embodiments described herein, but is defined in the claims.

## Claims

1. An absorbent article comprising a liquid pervious topsheet, a liquid impervious backsheet, and an absorbent core which comprises absorbent material and which is positioned between the liquid pervious topsheet and the liquid impervious backsheet; said absorbent article having a first and second longitudinal edge and a first and second transverse edge; wherein the absorbent article comprises a waist pocket positioned near the first transverse edge,
wherein the waist pocket comprises
a proximal transversal portion extending from a proximal transverse edge of the waist pocket, which is proximal to the first transverse edge, towards a distal transversal edge of the waist pocket opposite to the proximal transverse edge;
a distal transversal portion extending from the distal transverse edge of the waist pocket towards the proximal transverse edge of the waist pocket; and
a central transversal portion between the proximal transversal portion and the distal transversal portion and comprising a transversal center line of the waist pocket;
a first longitudinal side edge,
a second longitudinal side edge,
a first nonwoven layer and a second nonwoven layer;
wherein the proximal transversal portion is at least partially attached to a body facing side of the absorbent article and the distal transversal portion is at least partially unattached to the body facing side of the absorbent article, such that a waist pocket opening is formed between the distal transversal portion and the topsheet; and
wherein
the first nonwoven layer and/or the second nonwoven layer comprise, preferably consist of, a hydrophilic nonwoven.

2. The absorbent article of claim 1, wherein the waist pocket further comprising at least one elastic member positioned between the first and second nonwoven layer and extending substantially transversally between the first longitudinal side edge and the second longitudinal side edge.

3. The absorbent article of claim 1 or 2, wherein the first nonwoven layer and the second nonwoven layer are the same hydrophilic nonwoven, preferably wherein the first nonwoven layer and the second nonwoven layer are connected via a folded connection.

4. The absorbent article of claim 1 or 2, the first nonwoven layer and the second nonwoven layer are different nonwovens and wherein the second nonwoven is an inner nonwoven facing inwards, e.g. facing towards the topsheet, and wherein the first nonwoven is an outer nonwoven intended to face towards a wearer.

5. The absorbent article of any one of of the preceding claims,
wherein the hydrophilic nonwoven is chosen from a treated nonwoven, such a treated nonwoven comprising polyethylene (PE), polypropylene (PP), polyester or polyethylene terephthalate (PET) or combinations thereof; or
wherein the hydrophilic nonwoven is chosen from a nonwoven which substantially consist of a hydrophilic material(s); or
wherein the hydrophilic nonwovens are chosen from nonwovens comprising a mixture of hydrophobic polymers blended or grafted with hydrophilic additives.

6. The absorbent article of any one of the preceding claims, wherein the hydrophilic nonwoven is chosen from treated nonwoven which has been treated with a surface treatment chosen from a surface coating, such as a surfactant coating, a plasma treatment, a corona discharge treatment, preferably a surface coating.

7. The absorbent article of any one of the former claims, wherein the hydrophilic nonwoven is chosen from the group consisting of: a nonwoven which has been treated with a surfactant coating, a nonwoven which comprises a coating comprising hydrophilic agent, or nonwovens obtained by mixing a hydrophilic agent directly in a thermoplastic polymer resin before being processed into the nonwoven, e.g. via spunbonding, melt blowing, dry-laying, wet-laying, spunlacing or hydroentanglement.

8. The absorbent article of claim 4,
wherein the inner nonwoven is a nonwoven chosen from the group consisting of: a nonwoven which has been treated with a surfactant coating, a nonwoven which comprises a coating comprising hydrophilic agent, or nonwovens obtained by mixing a hydrophilic agent directly in a thermoplastic polymer resin before being processed into the nonwoven, e.g. via spunbonding, melt blowing, dry-laying, wet-laying, spunlacing or hydroentanglement.

9. The absorbent article of any one of the preceding claims 2 -8,
wherein the at least one elastic member of the waist pocket is positioned such that a central grammage of elastic material in the central transversal portion is lower than a distal grammage of elastic material in the distal transversal portion.

10. The absorbent article of any one of the preceding claims 2 - 9, wherein the distal transversal portion comprises a first distal transversal sub-portion extending from the distal transverse edge of the waist pocket and a second distal transversal sub-portion between the first transversal sub-portion and the central transversal portion, and wherein a first distal grammage of elastic material in the first distal transversal sub-portion is higher than a second distal grammage of elastic material in the second distal transversal sub-portion; and/or wherein the proximal transversal portion comprises a first proximal transversal sub-portion extending from the proximal transverse edge of the waist pocket and a second proximal transversal sub-portion between the first proximal transversal sub-portion and the central transversal portion, and wherein a first grammage of elastic material in the first proximal transversal sub-portion is higher than a second grammage of elastic material in the second proximal transversal sub-portion.

11. The absorbent article of any one of the preceding claims,
wherein each one of the proximal transversal portion and the distal transversal portion has a surface area which is substantially double of a surface area of the central transversal portion.

12. The absorbent article of any one of the preceding claims, wherein the absorbent article comprises a pair of longitudinally extending leg cuffs, wherein the first longitudinal side edge and the second longitudinal side edge of the waist pocket are adhesively attached to the leg cuffs.

13. The absorbent article of any one of the preceding claims, wherein the first and second nonwoven layer consist of an at least partially folded hydrophilic nonwoven sheet, preferably wherein the at least partially folded hydrophilic nonwoven sheet is folded according to a C-fold or L-fold configuration.

14. The absorbent article of the previous claim,
wherein a free portion of the at least partially folded nonwoven is facing the body facing side of the absorbent article, and wherein the free portion is hydrophilic.

15. The absorbent article of any one of the preceding claims,
wherein the first and/or second nonwoven layer comprises a treated spunbond nonwoven, a treated meltblown nonwoven, a treated spunlaced nonwoven or combination thereof.

16. The absorbent article of any one of the preceding claims, wherein the first and/or second nonwoven layer is a treated spunbond-meltblown-spunbond, SMS, nonwoven.

17. The absorbent article of any one of the preceding claims, wherein the first and/or second nonwoven layer has a grammage of between 4 - 15 gsm, preferably between 6 - 12 gsm, most preferably between 8 - 10 gsm.

18. The absorbent article of any one of the preceding claims, wherein the first and/or second nonwoven layer comprise, preferably consist of, any one of treated polypropylene, treated polylactic acid, and treated biobased polyethylene.

19. The absorbent article of any one the preceding claims, wherein the first and the second nonwoven layer consist of treated polypropylene.
